(19) Europäisches Patentamt
European Patent Office
Office européen des brevets

(11) Numéro de publication: **0 062 560**
**A1**

(12) # DEMANDE DE BREVET EUROPEEN

(21) Numéro de dépôt: **82400518.5**

(22) Date de dépôt: **23.03.82**

(51) Int. Cl.³: **C 07 D 453/02**, A 61 K 31/435

(30) Priorité: **03.04.81 FR 8106713**

(43) Date de publication de la demande: **13.10.82**
**Bulletin 82/41**

(84) Etats contractants désignés: **BE CH DE FR GB IT LI LU NL SE**

(71) Demandeur: **PHARMINDUSTRIE, 35 Quai du Moulin de Cage, F-92231 Gennevilliers (FR)**

(72) Inventeur: **Dubroeucq, Marie-Christine, 13, Villa Malleville, F-95880 Enghien-les-Bains (FR)**
Inventeur: **Rataud, Jean Ernest Marcel André, 115 rue Reuilly, F-75012 Paris (FR)**

(74) Mandataire: **Houssin, Jean, P C U K Produits Chimiques Ugine Kuhlmann Service Propriété Industrielle Tour *Manhattan - Cedex* 21, F-92087 Paris La Defense 2 (FR)**

(54) **Nouveaux dérivés de N,N-diméthyl (aza-1 bicyclo (2,2,2)octyl-3)-10 10H phénothiazinesulfonamide-2, procédé pour leur préparation et utilisation comme médicaments.**

(57) Composés de formule générale

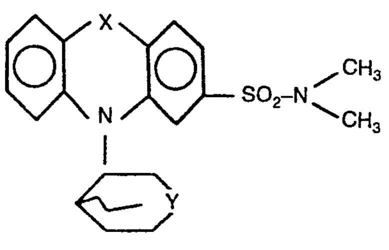

dans laquelle soit X représente un atome de soufre et Y représente le groupe N-oxyde soit X représente le groupe sulfinyle ou le groupe sulfonyle et Y représente un atome d'azote ou le groupe N-oxyde et leurs sels d'addition avec les acides minéraux ou organiques, leur procédé de préparation et leur utilisation comme médicament pour le traitement des ulcères gastriques et duodénaux.

EP 0 062 560 A1

Nouveaux dérivés de N,N-diméthyl [aza-1 bicyclo[2,2,2] octyl-3]-10 10H phénothiazinesulfonamide-2, procédé pour leur préparation et utilisation comme médicaments

L'invention a pour objet de nouveaux composés utilisables comme médicaments notamment pour le traitement des ulcères gastriques et duodénaux. Ils peuvent être représentés par la formule générale :

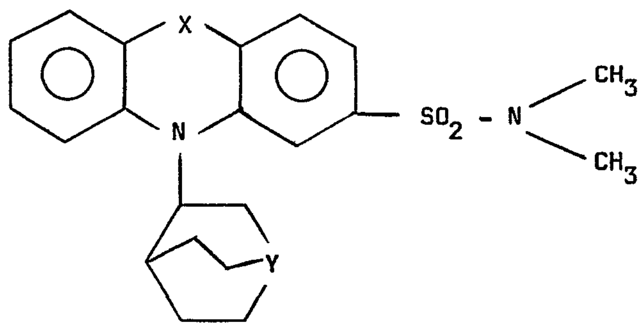

(I)

dans laquelle soit X représente un atome de soufre et Y représente le groupe N-oxyde soit X représente le groupe sulfinyle ou le groupe sulfonyle et Y représente un atome d'azote ou le groupe N-oxyde.

Lorsque X représente le groupe sulfinyle, la molécule des composés de formule (I) comporte deux centres asymétriques et donc, pour une signification donnée de Y, il y a deux diastéréoisomères correspondant à la formule (I).

Chacun de ces isomères, racémiques ou énantiomères et leurs mélanges font partie de l'invention de même que les sels d'addition de ces composés avec les acides minéraux ou organiques.

Les composés de formule (I) pour lesquels X représente le groupe sulfinyle ou le groupe sulfonyle et Y un atome d'azote peuvent être préparés par oxydation du composé de formule

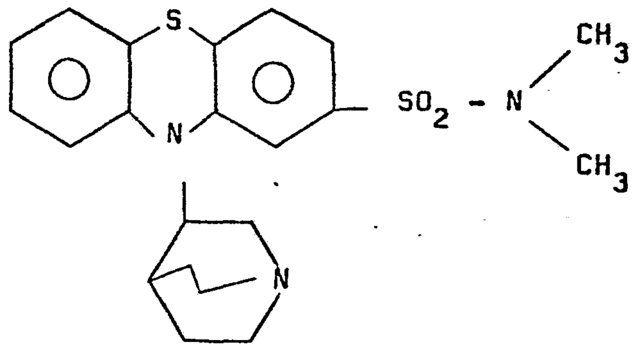

(II)

sous forme de sels, de préférence de sels dérivés d'acides forts tels que l'acide chlorhydrique, l'acide sulfurique ou l'acide méthanesulfonique.

L'oxydation est effectuée selon des méthodes connues en soi, qui permettent d'oxyder un sulfure en sulfoxyde ou en sulfone, par exemple celles décrites par J. MARCH, Advanced Organic Chemistry p. 112, Mc Graw-Hill 1977. Des méthodes avantageuses consistent à utiliser comme agent oxydant le métapériodate de sodium en milieu aqueux ou l'eau oxygénée dans l'eau et/ou l'acide acétique à une température comprise entre 20 et 100°C.

Pour obtenir préférentiellement les composés de formule (I) dans laquelle X représente le groupe sulfinyle, il est avantageux d'opérer à une température comprise entre 20 et 30°C avec comme agent oxydant le métapériodate de sodium en milieu aqueux.

Pour obtenir préférentiellement les composés de

formule (I) dans laquelle X représente le groupe sulfonyle il est avantageux d'opérer à une température comprise entre 50 et 100°C en présence d'un excès d'eau oxygénée en milieu aqueux, acétique ou hydroacétique.

Les composés de formule (I) pour lesquels Y représente le groupe N-oxyde peuvent être préparés par oxydation des composés de formule

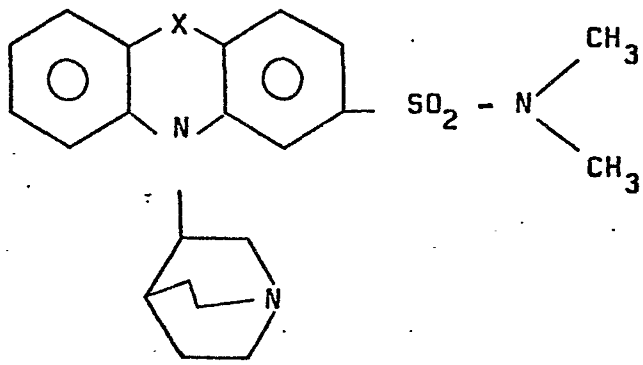

(III)

dans laquelle X a la même signification que dans la formule (I).

Pour réaliser cette oxydation on fait appel à des procédés connus en soi, par exemple ceux décrits par J. MARCH, Advanced Organic Chemistry p. 1111 Mc Graw-Hill 1977. La méthode la plus courante consiste à chauffer à l'ébullition le produit à oxyder dans une solution alcoolique d'eau oxygénée.

Les mélanges réactionnels obtenus par les procédés décrits précédemment sont traités selon des méthodes classiques, (évaporation, extraction à l'aide d'un solvant, distillation, cristallisation, chromatographie, etc...) afin d'isoler les composés de formule (I) à l'état pur.

Les composés de formule (I) sous forme de base libre peuvent éventuellement être transformés en sels d'addition avec un acide minéral ou organique par action d'un tel acide au sein d'un solvant approprié.

Le composé de formule (II) peut être préparé par le procédé décrit dans la demande de brevet français 81 00442.

Les exemples suivants illustrent l'invention sans la limiter.

EXEMPLE 1 : N,N-diméthyl [aza-1 bicyclo [2,2,2] octyl-3] -10 10H phénothiazinesulfonamide-2 S-oxyde (mélange des 2 diastéréoisomères)

A 33 ml d'une solution aqueuse d'acide méthanesulfonique 1,25 N on ajoute 300 ml d'eau, puis, sous agitation on introduit 17 g de N,N-diméthyl [aza-1 bicyclo [2,2,2] octyl-3] -10 10H phénothiazinesulfonamide-2. On ajoute à la solution obtenue une solution de 9,2 g de métapériodate de sodium dans 90 ml d'eau.

On agite 48 h à température ambiante. La solution est alcalinisée à l'aide d'ammoniaque concentré et extraite 3 fois avec 200 ml d'acétate d'éthyle. La phase organique est séchée sur sulfate de magnésium et évaporée. Le résidu obtenu est fixé sur une colonne de silice et on élue sous pression avec un mélange chloroforme 50/diéthylamine 10/hexane 40. On isole ainsi 9,5 g d'un mélange des diastéréoisomères de N,N-diméthyl [aza-1 bicyclo [2,2,2] octyl-3] -10 10H phénothiazinesulfonamide-2 S oxyde. L'analyse du mélange par chromatographie en phase liquide montre que le pourcentage de chaque diastéréoisomère est d'environ 50 %.

Ce mélange est mis en solution dans l'éthanol et

transformé par addition d'une solution éthanolique d'acide chlorhydrique en son monochlorhydrate qui est recristallisé dans un mélange éthanol 95/ eau 5.

Le composé obtenu fond à 250°. L'analyse du mélange par chromatographie en phase liquide montre que le pourcentage de chaque diastéréoisomère est alors de 82 % (forme A) et 18 % (forme B).

EXEMPLE 2 : N,N-diméthyl [aza-1 bicyclo [2,2,2] octyl-3] -10 10H phénothiazinesulfonamide-2 S-oxyde. (Forme A pure)

Du mélange des deux diastéréoisomères 50/50 préparé selon l'exemple 1, on isole, après une seconde chromatographie sous pression avec un mélange chloroforme 50/diéthanolamine 10/hexane 40, un des diastéréoisomères (forme A).

Ce composé est transformé au sein de l'éthanol en son monochlorhydrate. Après recristallisation dans un mélange éthanol 95/ eau 5, le produit fond à 260°C.

EXEMPLE 3 : N,N-diméthyl [aza-1 bicyclo [2,2,2] octyl-3] -10 10H phénothiazinesulfonamide-2 S-dioxyde

A une solution agitée de 4,1 g de N,N-diméthyl [aza-1 bicyclo [2,2,2] octyl-3] -10 10H phénothiazinesulfonamide-2 dans 15 ml d'acide acétique, on ajoute lentement une solution de 0,65 ml d'acide méthanesulfonique dans 15 ml d'acide acétique et enfin une solution de 2,5 ml d'eau oxygénée à 30 % dans 5 ml d'acide acétique.

Ce mélange est chauffé à 60° sous agitation pendant 4 heures, puis versé dans 300 ml d'eau. On alcalinise à l'aide d'ammoniaque concentré, puis extrait deux fois avec 100 ml

d'acétate d'éthyle. La phase organique est séchée sur sulfate de magnésium et évaporée sous pression réduite. Le résidu obtenu est fixé sur une colonne de silice, et on élue sous pression avec un mélange chloroforme 95/diéthylamine 5.

On isole ainsi 3,72 g de N,N-diméthyl [aza-1 bicyclo [2,2,2] octyl-3]-10 10H phénothiazinesulfonamide-2 S-dioxyde, que l'on transforme au sein de l'éthanol en son chlorhydrate fondant à 250°C après recristallisation dans un mélange hydro-alcoolique 5/95.

<u>EXEMPLE 4</u> : N,N-diméthyl [aza-1 bicyclo [2,2,2] octyl-3]-10 10H phénothiazinesulfonamide-2 N-oxyde

A une solution agitée de 8,3 g de N,N-diméthyl [aza-1 bicyclo [2,2,2] octyl-3]-10 10H phénothiazinesulfonamide-2 dans 100 ml d'éthanol on ajoute 2,4 ml d'eau oxygénée à 31,5 %. On agite 4 heures au reflux. Après refroidissement, on ajoute lentement 30 ml d'une solution aqueuse saturée de sulfite de sodium pour détruire l'excès de péroxydes. On évapore les solvants sous pression réduite, reprend le résidu à l'eau et amène à pH 12 par addition d'une solution concentrée d'hydroxyde de sodium. La phase aqueuse est extraite 2 fois avec 20 ml de chloroforme ; les extraits organiques sont alors séchés sur sulfate de magnésium et évaporés sous pression réduite.

Le résidu obtenu est fixé sur une colonne de silice et on élue sous pression avec un mélange acétate d'éthyle 14/ méthanol 4/diéthylamine 3. On obtient ainsi 8 g de N,N-diméthyl [aza-1 bicyclo [2,2,2] octyl-3]-10 10H phénothiazinesulfonamide-2 N-oxyde, que l'on transforme au sein de l'acétone par addition d'éther chlorhydrique en son chlorhydrate fondant à 245°C.

EXEMPLE 5 : N,N-diméthyl [aza-1 bicyclo [2,2,2] octyl-3] -10 10H phénothiazinesulfonamide-2 S-oxyde N-oxyde

A une solution de 4,8 g de N,N-diméthyl [aza-1 bicyclo [2,2,2] octyl-3] -10 10H phénothiazinesulfonamide-2 S-oxyde préparé selon l'exemple 1, dans 60 ml d'éthanol on ajoute 1,3 ml d'une solution à 31 % d'eau oxygénée. On agite au reflux pendant 48 h. Après refroidissement, on ajoute lentement 30 ml d'une solution aqueuse saturée de sulfite de sodium pour détruire l'excès de péroxydes. On évapore les solvants sous pression réduite, reprend le résidu à l'eau et amène à pH 12 par addition d'une solution concentrée d'hydroxyde de sodium.

La phase aqueuse est extraite 2 fois avec 20 ml de chloroforme ; les extraits organiques sont alors séchés sur sulfate de magnésium et évaporés sous pression réduite. Le résidu obtenu est fixé sur une colonne de silice et on élue avec un mélange acétate d'éthyle 14/ méthanol 4/diéthylamine 3.

On obtient ainsi 1 g d'un mélange des deux diastéréoisomères de N,N-diméthyl [aza-1 bicyclo [2,2,2] octyl-3] -10 10H phénothiazinesulfonamide-2 S-oxyde N-oxyde fondant vers 130°C.

## PROPRIETES PHARMACOLOGIQUES

L'activité antisécrétoire des composés selon l'invention de formule (I) a été étudiée sur l'hypersécrétion gastrique stimulée par la pentagastrine chez le chien porteur d'une poche de Heidenhain.

PROTOCOLE EXPERIMENTAL

Trois chiens "mongrels" porteurs d'une poche de Heidenhain, à jeun depuis 18 heures, reçoivent une perfusion veineuse de 4 mg/kg/heure de pentagastrine sous un volume de 30 ml/heure pendant 4 heures. La sécrétion de la poche est recueillie dans un flacon qui est changé toutes les 15 minutes. Une heure et demie après le début de la perfusion, le produit à étudier est administré par voie orale dans une gélule n° 000. La dose efficace 50 % (DE 50) est la dose qui diminue de 50 % le débit acide horaire de la 3ème ou 4ème heure de l'expérimentation par rapport au débit acide de la deuxième heure de la perfusion. Les doses sont exprimées en produit sous forme de base.

RESULTATS

| PRODUITS | DE 50 en mg/kg po |
|---|---|
| Cimétidine | 2,3 |
| Exemple 1 | 2,3 |
| Exemple 2 | 2,5 |
| Exemple 3 | 10 |

Les produits de l'invention sont donc de puissants inhibiteurs de sécrétion gastrique. En particulier les produits des exemples 1 et 2 sont aussi actifs que la Cimétidine.

PROPRIETES TOXICOLOGIQUES

Les toxicités aiguës des composés selon l'invention ont été déterminées chez la souris mâle $CD_1$ (Charles RIVER)

par voie orale. Les $DL_{50}$ ont été calculées après 3 jours d'observation, par la méthode cumulative de J.J. REED et H. MUENCH. (Amer. J. Hyg. 1938, 27, 493).

Les composés se comportent comme des substances relativement peu toxiques chez la souris, puisque les $DL_{50}$ des composés se situent entre 500 et 1000 mg/kg.

## UTILISATION THERAPEUTIQUE

Les composés selon l'invention et leurs sels pharmaceutiquement acceptables peuvent être utilisés en thérapeutique humaine, sous forme de comprimés, capsules, gélules, suppositoires, solutions ingérables ou injectables, etc... comme inhibiteurs de sécrétion gastrique pour le traitement des ulcères duodénaux et gastriques.

La posologie dépend des effets recherchés et de la voie d'administration utilisée. Par exemple, par voie orale, elle peut être comprise entre 50 et 500 mg de substance active par jour, avec des doses unitaires allant de 10 à 100 mg.

Revendications de brevet

1. Composés de formule générale (I) :

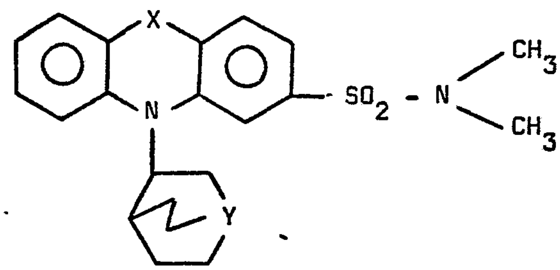

(I)

dans laquelle soit X représente un atome de soufre et Y le groupe N-oxyde soit X représente le groupe sulfinyle ou le groupe sulfonyle et Y un atome d'azote ou le groupe N-oxyde et leurs sels d'addition avec les acides minéraux ou organiques.

2. Composés de formule générale (I) dans laquelle X représente le groupe sulfinyle et Y un atome d'azote ou le groupe N-oxyde sous forme de leurs diastéréoisomères racémiques ou énantiomères et leurs sels d'addition avec les acides minéraux ou organiques.

3. Procédé de préparation des composés de formule (I) dans laquelle X représente le groupe sulfinyle ou le groupe sulfonyle et Y un atome d'azote caractérisé en ce que l'on oxyde des sels du composé de formule (II) :

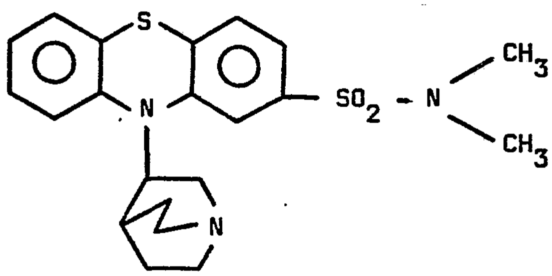

(II)

4. Procédé selon la revendication 3 caractérisé en ce que l'on utilise des sels dérivés d'acides forts tels que l'acide chlorhydrique, l'acide sulfurique ou l'acide méthanesulfonique.

5. Procédé selon les revendications 3 ou 4 caractérisé en ce que l'on oxyde au moyen du métapériodate de sodium en milieu aqueux à une température de 20 à 100°C.

6. Procédé selon la revendication 5 caractérisé en ce que l'on opère à une température de 20 à 30°C.

7. Procédé selon les revendications 3 ou 4 caractérisé en ce que l'on oxyde au moyen d'eau oxygénée dans l'eau, l'acide acétique ou un mélange de ces solvants à une température de 20 à 100°C.

8. Procédé selon la revendication 7 caractérisé en ce que l'on opère de 50 à 100°C.

9. Procédé de préparation des composés de formule (I) dans laquelle Y représente le groupe N-oxyde et X un atome de soufre, le groupe sulfinyle ou le groupe sulfonyle caractérisé en ce que l'on oxyde le composé de formule (III) :

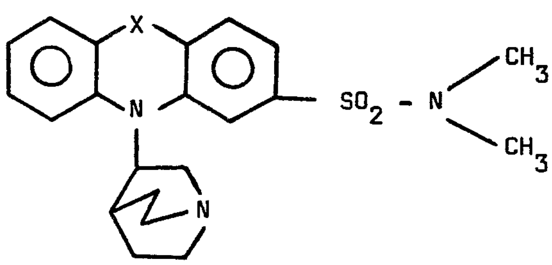

(III)

dans laquelle X a la même signification que dans la formule (I).

10. Procédé selon la revendication 9 caractérisé en ce que l'on oxyde le composé de formule (III) au moyen d'une solution alcoolique d'eau oxygénée à la température d'ébullition.

11. Médicaments caractérisés en ce qu'ils contiennent, en tant que principe actif, un composé selon les revendications 1 ou 2.

12. Médicaments selon la revendication 11 sous forme de doses unitaires contenant 10 à 100 mg de principe actif.